# FASCICULE DE BREVET EUROPEEN

(11) **EP 1 237 474 B1**
(45) Date de publication et mention de la délivrance du brevet: **08.03.2006**
(21) Numéro de dépôt: 00988922.1
(22) Date de dépôt: 14.12.2000
(51) Int. Cl.: A61B 5/0408, G01N 27/403

(54) **MICROELECTRODE SUPPORT DE CELLULES A MEMBRANE EXCITABLE**
MIKROELEKTRODE ZUM TRAGEN VON ZELLEN MIT REIZBARER MEMBRAN
MICROELECTRODE SUPPORTING CELL WITH EXCITABLE MEMBRANE

(30) Priorité: 14.12.1999 FR 9915772
(43) Date de publication de la demande: 11.09.2002
(73) Titulaire: Microvitae Technologies, 13790 Peynier (FR)
(72) Inventeur: HERVE, Thierry, 38700 Le Sappey en Chartreuse (FR)
(74) Mandataire: de Beaumont, Michel
(86) Numéro de dépôt international: PCT/FR2000/003543
(87) Numéro de publication internationale: WO 2001/043636

(56) Documents cités:
- EP-A- 1 218 736
- WO-A-01/25769
- DE-A- 19 712 309

## Description

La présente invention concerne un détecteur de glycémie, et plus particulièrement une électrode de très petites dimensions susceptible de détecter l'activité électrique d'une cellule ou d'un amas de cellules à membrane excitable, par exemple des neurones, des cellules nodales autonomes du coeur ou des îlots de Langerhans.

De telles électrodes sont connues dans la technique et ont en particulier été décrites dans le brevet américain 5513636 de la société israélienne CB-Carmel Biotechnology Ltd.

Ce brevet décrit une électrode du type de celle représentée en figure 1 qui comprend une plaquette support 1 en un matériau isolant rigide tel que du silicium ou du verre. Sur cette plaquette est disposée une électrode constituée d'une couche conductrice 2 reliée à un fil 3. Cette couche conductrice a par exemple une épaisseur inférieure à 0,1 µm. L'ensemble de la plaquette 1 est recouvert d'une couche isolante 4 munie d'une ouverture 5 en regard de la couche conductrice 2. Une cellule, par exemple un îlot de Langerhans, est disposée sur l'ouverture 5 de façon à adhérer à la couche conductrice 2 et aux parois de la couche isolante 4. Il est précisé que "la surface supérieure exposée de la couche isolante sur laquelle les cellules doivent être amenées à croître au moins partiellement (une partie de chaque cellule croît sur la plaque conductrice et une partie de la cellule croît sur la couche isolante) doit être traitée pour que les cellules croissent sur cette couche et tendent à y adhérer de façon forte et étanche" (colonne 4, lignes 14 à 18). Il est également précisé que "la forte adhérence et le scellement entre la cellule et la couche isolante empêchent le signal électrique en provenance de la cellule d'être atténué par un court-circuit entre la cellule ou la plaque conductrice et le milieu environnant la cellule" (colonne 4, lignes 22 à 26).

Ainsi, dans ce brevet, il est prévu que la cellule ou l'îlot de Langerhans est très proche de l'électrode correspondante ou en contact avec celle-ci. Il est également prévu que la partie du fluide environnant piégée entre la cellule et l'électrode est située dans une enceinte close par les parois de l'ouverture 5, la couche conductrice 2 et la cellule 7.

En outre, ce document prévoit que plusieurs structures telles que celle illustrée en coupe en figure 1 peuvent être disposées en parallèle sur une même plaquette : les ouvertures 5 sont côte à côte et les fils 3 sont parallèles les uns aux autres, dans une direction perpendiculaire à la direction d'alignement des cellules.

La demande de brevet allemand DE-A-197 12 309 vise une microélectrode pour des cellules de petit diamètre (10 *µ*m) dans laquelle on recherche également un contact entre cellules et électrodes. Les caractéristiques du préambule de la revendication 1 sont divulgées dans ce document.

La demanderesse a constaté qu'en pratique les signaux électriques détectés par de telles électrodes n'étaient pas optimaux et a cherché à augmenter la détectivité des électrodes.

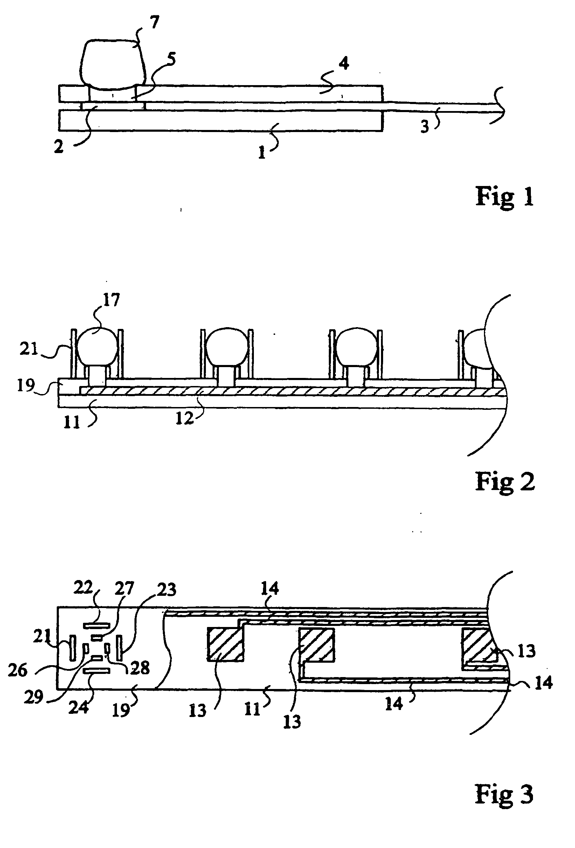

Par ailleurs, la structure illustrée en figure 1 est destinée à être incorporée dans une capsule. Lors de cette incorporation, il apparaît en pratique qu'au moins une partie des îlots de Langerhans se décrochent de l'ouverture au-dessus de laquelle ils doivent être situés ou s'agrègent entre eux.

Un objet selon la présente invention est de pallier les inconvénients de cette électrode antérieure et de prévoir une électrode à seuil de détectivité plus élevé.

Pour atteindre ces objets, la présente invention prévoit une microélectrode destinée à porter au moins une cellule à membrane excitable, comprenant une plaquette isolante munie d'ouvertures, chaque ouverture débouchant sur une électrode de détection et étant entourée de murs en matériau isolant sensiblement perpendiculaires à la plaquette pour bloquer en position ladite au moins une cellule, des moyens d'écartement étant prévus pour maintenir ladite au moins une cellule à une distance déterminée de l'électrode correspondante.

Selon un mode de réalisation de la présente invention, les moyens d'écartement sont des murs en matériau isolant, sensiblement perpendiculaires à la plaquette.

Selon un mode de réalisation de la présente invention, les murs sont formés à partir d'une structure multicouche de matériau isolant.

Selon un mode de réalisation de la présente invention, la structure multicouche est formée par des étapes successives de dépôt à la tournette et de recuit d'un polyimide.

Selon un mode de réalisation de la présente invention, ladite ouverture est fermée par une plaquette isolante sur laquelle repose l'électrode.

Selon un mode de réalisation de la présente invention, ladite au moins une cellule à membrane excitable est un îlot de Langerhans.

Selon un mode de réalisation de la présente invention, plusieurs microélectrodes sont empilées, les murs en matériau isolant servant d'entretoises entre deux électrodes superposées.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
la figure 1 est une vue en coupe partielle et schématique d'une électrode selon l'art antérieur ;
la figure 2 est une vue en coupe schématique et partielle d'une électrode selon un mode de réalisation de la présente invention ;
la figure 3 est une vue de dessus schématique de l'électrode de la figure 2 ;
la figure 4 est une vue en coupe partielle illustrant un autre mode de réalisation ne faisant pas partie de la présente invention ;
la figure 5 est une vue en coupe partielle illustrant un autre mode de réalisations ne faisant pas partie de la présente invention ; et
la figure 6 est une vue en coupe partielle illustrant un autre mode de réalisation ne faisant pas partie de la présente invention.

Dans les diverses figures, les épaisseurs des diverses couches et supports ne sont pas à l'échelle et pourront être choisies de façon classique par l'homme du métier.

La figure 2 est une vue en coupe représentant de façon schématique et partielle un premier mode de réalisation de la présente invention. On part d'une plaquette support isolante 11, par exemple un film de faible épaisseur (quelques µm), sur laquelle on dépose, par exemple par évaporation, une couche mince d'un conducteur, couramnent un métal, par exemple de l'or, convenablement gravée pour définir des plaques conductrices formant électrodes et des pistes de liaison. Dans la partie droite de la vue de dessus partiellement écorchée de la figure 3, on peut voir un exemple de plaques conductrices 13 et de pistes de liaison 14. On dépose ensuite, par exemple par dépôt à la tournette, une couche isolante 19. Cette technique permet de déposer des couches ayant des épaisseurs de quelques µm à quelques dizaines de *µ*m selon la vitesse de rotation de la tournette. On pourra par exemple déposer une couche de polyimide mélangé à un solvant et ensuite recuit. La couche isolante 19 est munie d'ouvertures au-dessus des emplacements des électrodes détectrices, correspondant à des sites de positionnement d'îlots de Langerhans 17.

Selon un aspect de la présente invention, chaque site de dépôt de cellules, par exemple d'îlot de Langerhans, est encadré de moyens de blocage latéraux, par exemple quatre murs verticaux 21-24. L'espacement entre ces murs est choisi pour être supérieure ou égale à la valeur moyenne du diamètre de la ou des cellules considérées, cette valeur moyenne étant généralement de l'ordre de 100 µm pour un îlot de Langerhans. Les murs 21-24 pourront être réalisés par dépôt en couche épaisse, ou de préférence par dépôts successifs, d'un isolant et gravure. Ils auront par exemple une hauteur légèrement inférieure au diamètre d'un îlot de Langerhans. La gravure peut par exemple être réalisée par gravure ionique réactive (RIE) en présence d'O₂ ou de CHF₃ ou par laser excimère, ce qui permet de graver de façon sensiblement verticale des couches ayant des épaisseurs de l'ordre de 100 µm. Comme cela se voit en figure 3, les murs sont "ajourés" c'est-à-dire qu'ils ne forment pas un contour continu. Dans l'exemple représenté, ils ne se rejoignent pas au niveau des coins du carré qu'ils délimitent. Ceci est destiné à permettre à des nutriments (le milieu dans lequel le dispositif va être placé) d'atteindre toutes les cellules de l'îlot de Langerhans.

Selon un deuxième aspect de la présente invention, il est prévu des moyens d'espacement pour amener la partie basse de chaque îlot de Langerhans à se trouver à une hauteur sensiblement constante par rapport à l'électrode détectrice correspondante. En effet, selon cet aspect de la présente invention, il a été déterminé que l'on augmente de façon importante la tension détectée par une électrode quand la distance entre la partie basse d'un îlot de Langerhans et l'électrode détectrice correspondante a une valeur déterminée, faible mais non nulle. On pourra choisir une distance de l'ordre de la moitié du diamètre de l'Îlot de Langerhans, par exemple comprise entre 0, 2 et 0, 7 fois le diamètre de l'îlot de Langerhans, bien que d'autres valeurs soient possibles. Pour d'autres cellules ou groupes de cellules on pourra de même choisir une distance optimale.

Dans le cas des figures 2 et 3, ces moyens d'espacement sont constitués de petits murs 26-29, sur les sommets desquels s'appuie l'îlot de Langerhans.

Les matériaux constitutifs des éléments de l'électrode illustrée en figures 2 et 3 seront choisis pour permettre une fabrication simple et être biocompatibles. Par exemple, les divers matériaux isolants seront des polyimides tels que le polyimide PI 2611 de Dupont de Nemours et les matériaux conducteurs des couches d'or.

Comme le représente la figure 3, sur une même plaquette d'électrodes, on prévoira plusieurs sites disposés les uns derrière les autres dans le sens de la longueur de la plaquette. Ceci conduit à la réalisation d'un ensemble d'électrodes de petite dimension et particulièrement simple à implanter dans une capsule destinée à être placée dans le corps d'un patient. Bien que, pour clarifier la représentation, les cellules soient représentées comme espacées d'une distance supérieure à leur diamètre, on pourra prévoir que les murs sont disposés de telle sorte que deux cellules voisines sont très proches l'une de l'autre, les murs permettant d'éviter que deux cellules voisines ne s'aggrippent l'une à l'autre.

Selon un aspect de la présente invention, on pourra prévoir des murs de séparation plus hauts que le diamètre d'une cellule et empiler plusieurs microélectrodes, les murs de séparation servant d'entretoises entre deux microélectrodes superposées.

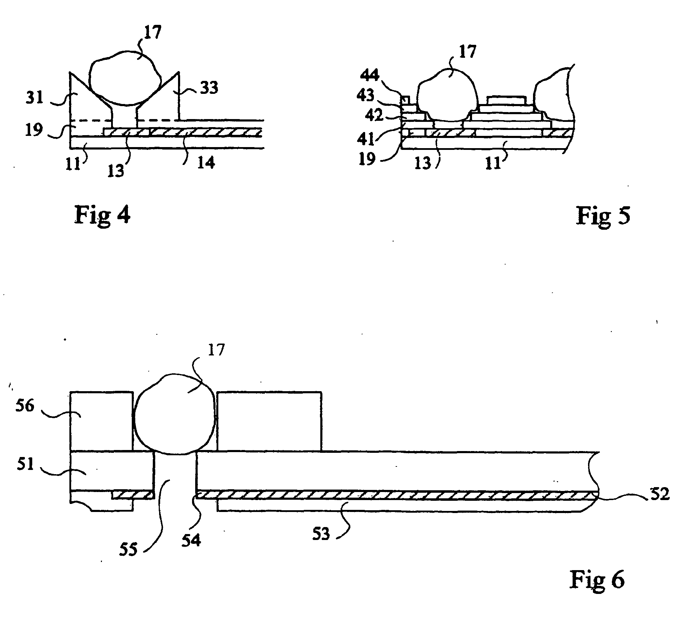

Les figures 4 à 6 représentent diverses variantes de réalisation d'un site d'électrode détectrice.

Dans les modes de réalisation des figures 4 et 5, l'ensemble des murs de blocage latéraux et des murs d'espacement est constitué d'une seule et même structure.

Dans le cas de la figure 4, les murs 21-24 sont remplacés par des murs 31-34 (seuls les murs 31 et 33 sont visibles dans la vue en coupe) . Le mur 31 assure les fonctions du mur de blocage latéral 21 et du mur d'espacement 26. De même, le mur 33 assure les fonctions du mur de blocage latéral 23 et du mur d'espacement 28. A cette fin, les murs 31 et 33 sont relativement larges et ont des faces supérieures inclinées vers l'intérieur.

La figure 5 représente une structure qui est globalement identique à celle de la figure 4 mais qui résulte d'un dépôt de couches multiples qui sont successivement gravées de façon appropriée pour former les plans inclinés en escalier. Ainsi, on dépose une succession de couches 41, 42, 43, 44 ..., par exemple une dizaine de couches successives ayant chacune une épaisseur de l'ordre de quelques µm à quelques dizaines de µm, chaque couche en un premier matériau étant recouverte d'une couche très mince d'un deuxième matériau servant d'arrêt de gravure. On procède ensuite à des gravures successives selon des fenêtres concentriques de plus en plus étroites pour obtenir les structures en escalier représentées. Inversement, on pourra après chaque dépôt procéder à une gravure, les couches successives étant gravées pour former des ouvertures plus larges que des ouvertures précédentes, et centrées sur celles-ci.

La figure 6 représente un autre mode de réalisation qui diffère des modes de réalisation précédents essentiellement du fait que la couche conductrice constituant l'électrode détectrice n'est pas placée au fond d'une ouverture correspondant à un trou borgne, mais sur la face opposée par rapport à l'îlot de Langerhans d'une ouverture traversante. Dans le mode de réalisation de la figure 6, on part d'un support relativement épais mais souple 51 dont la face inférieure est revêtue d'une métallisation 52. Sur cette métallisation est déposée une couche isolante de protection 53. La couche 53 est éliminée localement pour laisser apparaître une zone d'électrode détectrice 54 au niveau de chaque site de détection. Au niveau de chacun de ces sites, la plaquette support 51 est ouverte par un trou traversant 55 de sorte que l'électrode détectrice 54 constitue un anneau périphérique à l'ouverture du côté de la face inférieure de la plaquette 51. Dans ce mode de réalisation, l'épaisseur de la plaquette 51 constitue le moyen d'écartement fixant la distance entre la partie inférieure de l'îlot de Langerhans 17 et l'électrode détectrice 54. Du côté de la face supérieure est déposée une couche épaisse de matériau isolant 56 comportant des ouvertures plus larges que les ouvertures 55 au niveau de chaque site de détection. Cette couche 56 et les ouvertures correspondantes correspondent au moyen de blocage latéral décrit précédemment.

Dans le mode de réalisation de la figure 6, on notera que, du fait que l'ouverture 55 est une ouverture traversante, des nutriments peuvent arriver du côté inférieur de l'îlot de Langerhans par cette ouverture. Ainsi, il n'est pas nécessaire que le mur 56 bloquant latéralement l'îlot de Langerhans soit ajouré. On pourra prévoir, par exemple, une ouverture circulaire dans une couche épaisse.

## Revendications

1. Microélectrode destinée à porter au moins une cellule à membrane excitable immergée dans un fluide, comprenant une plaquette support isolante (11) et une couche isolante (19) munie d'ouvertures, chaque ouverture débouchant sur une électrode de détection (13) et étant entourée de murs (21-24) en matériau isolant sensiblement perpendiculaires à la plaquette pour bloquer en position ladite au moins une cellule, **caractérisée en ce qu'**elle comprend en outre des moyens d'écartement (26-29) pour maintenir ladite au moins une cellule à une distance déterminée de l'électrode correspondante, ladite distance étant occupée par ledit fluide.

2. Microélectrode selon la revendication 1, **caractérisée en ce que** les moyens d'écartement sont des murs en matériau isolant, sensiblement perpendiculaires à la plaquette.

3. Microélectrode selon la revendication 1, **caractérisée en ce que** les murs sont formés à partir d'une structure multicouche de matériau isolant.

4. Microélectrode selon la revendication 3, **caractérisée en ce que** la structure multicouche est formée par des étapes successives de dépôt à la tournette et de recuit d'un polyimide.

5. Microélectrode selon la revendication 1, **caractérisée en ce que** ladite ouverture est fermée par une plaquette isolante sur laquelle repose l'électrode.

6. Microélectrode selon la revendication 1, **caractérisée en ce que** ladite au moins une cellule à membrane excitable est un îlot de Langerhans.

7. Empilement de microélectrodes selon l'une quelconque des revendications 1 à 6, dans lequel les murs en matériau isolant pour bloquer en position ladite au moins une cellule servent d'entretoises entre deux microélectrodes superposées.

## Claims

1. A microelectrode intended to support at least one cell with an excitable membrane immerged in a fluid, including an insulating support plate (11) and an insulating layer (19) provided with openings, each opening emerging on a detection electrode (13) and being surrounded with walls (21-24) made of insulating material, substantially perpendicular to the plate to block in position said at least one cell, **characterized in that** it further includes spacing means (26-29) for maintaining said at least one cell at a determined distance from the corresponding electrode, said distance being occupied by said fluid.

2. The microelectrode of claim 1, **characterized in that** the spacing means are walls made of an insulating material, substantially perpendicular to the plate.

3. The microelectrode of claim 1, **characterized in that** the walls are formed from a multiple-layer structure of insulating material.

4. The microelectrode of claim 3, **characterized in that** the multiple-layer structure is formed by successive steps of spin-on deposition and of anneal of a polyimide.

5. The microelectrode of claim 1, wherein said opening is closed by an insulating plate on which the electrode is laid.

6. The microelectrode of claim 1, **characterized in that** said at least one cell with an excitable membrane is an islet of Langerhans.

7. A stack of the microelectrodes of any of claims 1 to 6, in which the walls of insulating material to block in position said at least one cell are used as spacers between two superposed microelectrodes.

## Patentansprüche

1. Mikroelektrode zur Aufnahme wenigstens einer in ein Strömungsmittel eingetauchten Zelle mit einer anregbaren Membran, welche eine isolierende Tragplatte bzw. -plakette (11) und eine mit Öffnungen versehene isolierende Schicht (19) aufweist, wobei jede Öffnung über einer Detektionselektrode (13) mündet und von Mauern bzw. Wandungen (21 - 24) aus einem isolierenden Material umgeben ist, die im wesentlichen senkrecht zu der Plakette sind, um die wenigstens eine Zelle in ihrer Stellung zu blockieren bzw. zu fixieren, **dadurch gekennzeichnet, daß** die Mikroelektrode des weiteren Abstandsmittel (26 - 29) aufweist, um die genannte wenigstens eine Zelle in einem vorgegebenen Abstand von der entsprechenden Elektrode zu halten, wobei dieser Abstand von dem genannten Strömungsmittel eingenommen wird.

2. Mikroelektrode nach Anspruch 1, **dadurch gekennzeichnet, daß** die Abstandsmittel Mauern bzw. Wandungen aus einem isolierenden Material im wesentlichen senkrecht zu der Plakette sind.

3. Mikroelektrode nach Anspruch 1, **dadurch gekennzeichnet, daß** die Mauern bzw. Wandungen ausgehend von einer Mehrschicht-Struktur aus isolierendem Material gebildet sind.

4. Mikroelektrode nach Anspruch 3, **dadurch gekennzeichnet, daß** die Mehrschichtstruktur mittels aufeinanderfolgender Stufen von Schleuder-Abscheidung und Glühvergütung eines Polyimids gebildet werden.

5. Mikroelektrode nach Anspruch 1, **dadurch gekennzeichnet dass** die genannte Öffnung durch eine isolierende Plakette geschlossen ist, auf welcher die Elektrode aufruht.

6. Mikroelektrode nach Anspruch 1, **dadurch gekennzeichnet, daß** die genannte wenigstens eine Zelle mit anregbarer Membran eine Langerhans-Insel ist.

7. Stapel von Mikroelektroden gemäß einem der Ansprüche 1 bis 6, bei welchem die Mauern bzw. Wandungen aus isolierendem Material zur Lageblockierung bzw.-fixierung der wenigstens einen Zelle als Zwischenabstandslage zwischen zwei übereinander liegenden Mikroelektroden dienen.
